# EUROPEAN PATENT SPECIFICATION

(11) **EP 4 313 940 B1**
(45) Date of publication and mention of the grant of the patent: **27.11.2024**
(21) Application number: 22717581.7
(22) Date of filing: 23.03.2022
(51) Int. Cl.: C07C 251/08, C07C 249/02, C07C 251/06

(54) **NOVEL IMINE**
NEUES IMIN
NOUVELLE IMINE

(30) Priority: 29.03.2021 GB 202104415
(43) Date of publication of application: 07.02.2024
(73) Proprietor: Syngenta Crop Protection AG, 4058 Basel (CH)
(72) Inventor: HODGES, George Robert, Bracknell, RG42 6EY (GB)
(74) Representative: SYNGENTA IP
(86) International application number: PCT/EP2022/057619
(87) International publication number: WO 2022/207427

(56) References cited:
- BETZ J ET AL: "A New @a-Selective Synthetic Equivalent for the Crotyl Anion in Additions to Imines", TETRAHEDRON LETTERS, ELSEVIER, AMSTERDAM , NL, vol. 36, no. 23, 5 June 1995 (1995-06-05), pages 4043 - 4046, XP004027911, ISSN: 0040-4039, DOI: 10.1016/0040-4039(95)00670-8

## Description

The present technology relates to a novel imine, N-(2-methoxy-1-methyl-ethyl)hexan-3-imine, its isomers, and the production thereof.

The present technology is useful in production of chemicals known as starting materials for herbicides the S-Metolachlor (*S*-MOC) and metolachlor. *S*-Metolachlor (*S*-MOC) and metolachlor are part of the chloroacetanilide family of herbicides, used to control grasses and broad-leafed weeds in maize. *S-*Metolachlor and metolachlor are known to be produced by reacting (*S*)-NAA or NAA and with chloroacetyl chloride. Betz, J. A New alpha-Selective Synthetic Equivalent for the Crotyl Anion in Additions to Imines, TETRAHEDRON LETTERS, 36(23), (1995), pages 4043-4046 discloses the synthesis of structurally related compounds

However, there is a need for alternative routes to produce S-MOC. The present invention therefore defines a novel imine compound of formula (I)

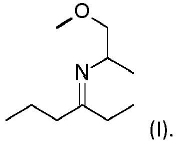

Production of (*S*)-NAA using the novel imine of the present technology is shown below:

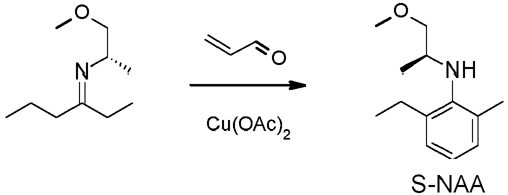

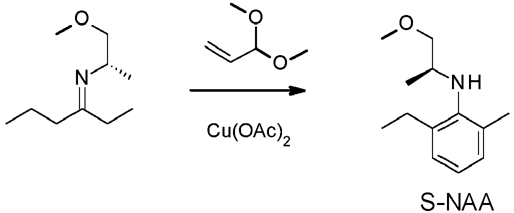

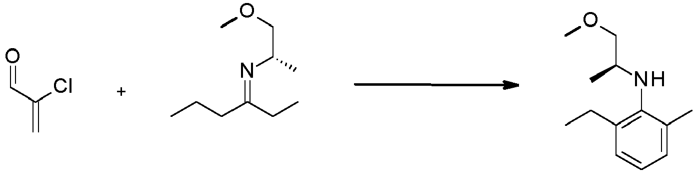

Preferably the compound of formula (I) is the isomer of formula (Ia)

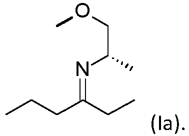

The present invention provides a method producing a compound of formula (I) 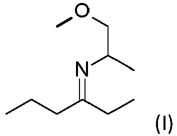 the method comprising: reacting a compound of formula (II) 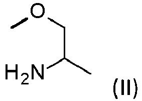 with a compound of formula (III)

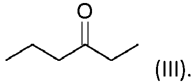

The present invention also provides a method of producing a compound of formula (Ia) 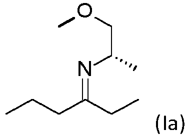 the method comprising: reacting a compound of formula (IIa) 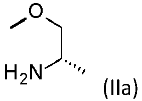 with a compound of formula (III)

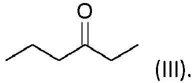

The methods are shown below in Scheme 1:

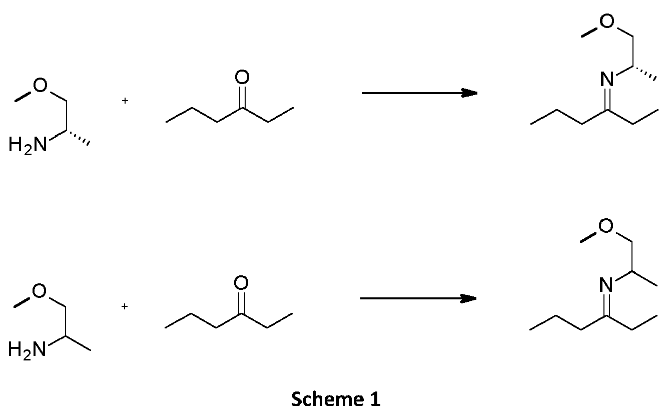

The invention is described by the following non-limiting Examples.

### Examples

### Experiment 1

An oven dried 3-necked round bottomed flask equipped with thermometer and magnetic follower was purged with nitrogen. A Dean-Stark trap equipped with condenser (filled to the arm of the trap with molecular sieves and reaction solvent) was attached. Foil was used to insulate the system and so minimise heat loss in the Dean-Stark apparatus.

Toluene (150 mL, ca. 60% of reactor volume) was added. The ketone (56.09 mmol, approx 5% v/v) and amine (112.18 mmol, 2.0 eq) were then charged through a seal via syringe. The reaction was stirred at ambient temperature for 10 mins before addition of TFA (0.01 eq), then stirred for a further 10 mins at ambient before heating to 110 °C (internal temperature). The reaction was sampled and monitored by GC.

The vessel was cooled to ambient, and the solvent removed *in vacuo* (35 mbar, 30 °C). Acetonitrile was added to the crude product and then removed *in vacuo* (2 repeats) to remove residual toluene and afford the chiral imine (S)-N-(2-Methoxy-1-methyl-ethyl)hexan-3-imine as a yellow liquid, 7.85 g, 82%, 93.4% strength, >99% e.r.

### Experiment 2

An oven dried 3 necked round-bottomed flask equipped with thermometer was purged with inert gas. A Dean stark trap equipped with condenser (filled with molecular sieves and reaction solvent filled to the arm of the trap) was attached.

Solvent (10-150 mL, toluene) was added. The ketone (2-75 mmol) and amine (4-150 mmol, 2.0 eq) were then charged through seal. Reaction stirred at room temperature for 10 min before addition of acid 0.01-1.0 eq). The reaction was stirred at room temperature for 10 mins before heating to 105 °C internal temperature. The reaction was observed to be at steady state for 30 min before stirring at temperature for the reaction time. Reaction may be sampled into GC vials.

The vessel was cooled to ambient, either A: the solution neutralised with potassium carbonate if necessary, filtered and the product extracted in toluene if necessary (3 x 100 mL) or B: addition of water followed by phase separation via liquid liquid extraction or phase separator membrane. The solvent was then dried followed by azeotroping with acetonitrile to remove residual toluene.

The invention is defined by the claims.

## Claims

1. A compound of formula (I)

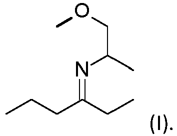

2. The compound of claim 1, where the compound of formula (I) is the isomer of formula (Ia)

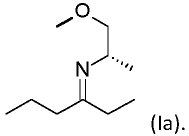

3. A method producing a compound of formula (Ia) 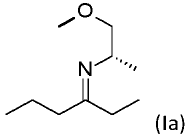 the method comprising: reacting a compound of formula (IIa) 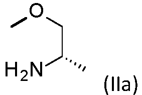 with a compound of formula (III)

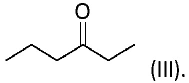

4. A method producing a compound of formula (I) 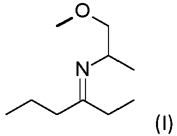 the method comprising: reacting a compound of formula (II) 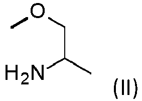 with a compound of formula (III)

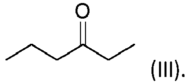

## Patentansprüche

1. Verbindung der Formel (I)

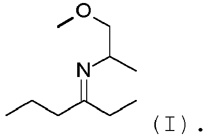

2. Verbindung nach Anspruch 1, wobei es sich bei der Verbindung der Formel (I) um das Isomer der Formel (Ia) handelt:

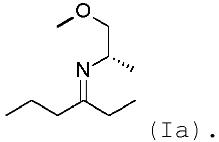

3. Verfahren zur Herstellung einer Verbindung der Formel (Ia) 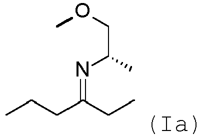 wobei das Verfahren Folgendes umfasst: Umsetzen einer Verbindung der Formel (IIa) 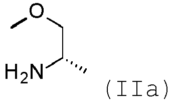 mit einer Verbindung der Formel (III)

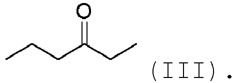

4. Verfahren zur Herstellung einer Verbindung der Formel (I) 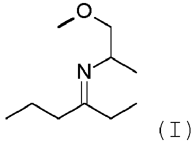 wobei das Verfahren Folgendes umfasst: Umsetzen einer Verbindung der Formel (II) 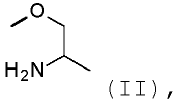 mit einer Verbindung der Formel (III)

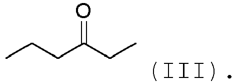

## Revendications

1. Composé de formule (I)

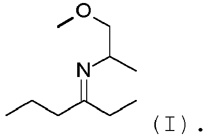

2. Composé selon la revendication 1, où le composé de formule (I) est l'isomère de formule (Ia)

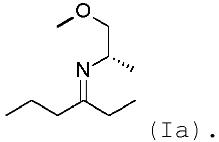

3. Procédé produisant un composé de formule (Ia) 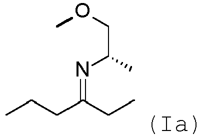 le procédé comprenant : la mise en réaction d'un composé de formule (IIa) 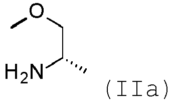 avec un composé de formule (III)

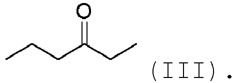

4. Procédé produisant un composé de formule (I) 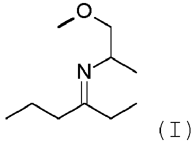 le procédé comprenant : la mise en réaction d'un composé de formule (II) 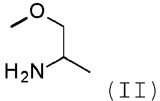 avec un composé de formule (III)
